# EUROPEAN PATENT APPLICATION

(11) **EP 3 662 954 A1**
(43) Date of publication of application: **10.06.2020**
(21) Application number: 17920398.9
(22) Date of filing: 02.08.2017
(51) Int. Cl.: A61M 25/00

(54) **CATHETER**

(71) Applicant: Asahi Intecc Co., Ltd., Seto-shi, Aichi 489-0071 (JP)
(72) Inventor: SUGAWARA Kei, Seto-shi, Aichi 489-0071 (JP)
(74) Representative: Winter, Brandl, Fürniss, Hübner, Röss, Kaiser, Polte - Partnerschaft mbB
(86) International application number: PCT/JP2017/028140
(87) International publication number: WO 2019/026220

(57) **Abstract**

[Problem] Provided is a catheter of which a tip can be downsized with a decrease of torquability being reduced and in which a joining strength between the tip and a catheter shaft can be further enhanced.

[Solution] A catheter 1 includes a catheter shaft 20 and a tip 30 made of a resin. The catheter shaft 20 has a coil body 22 having a first portion 22a with a first inner diameter, a tapered portion disposed at a distal end of the first portion 22a having a diameter decreasing from the first inner diameter to a second inner diameter and arranged in the inside of the tip 30; an inner layer 24 covering an inner peripheral surface of the first portion 22a; and an outer layer 28 covering an outer peripheral surface of the first portion 22a. The tip 30 has an outer-layer joining region 30a joined to a distal end 28a of the outer layer 28, and an inner-layer joining region 30b joined to a distal end 24a of the inner layer 24. A gap 50 is disposed between adjacent element wires at the tapered portion 22b, and the outer-layer joining region 30a is integrated with the inner-layer joining region 30b through the gap 50.

## Description

### TECHNICAL FIELD

The present invention relates to a catheter including a catheter shaft and a resin tip.

### BACKGROUND ART

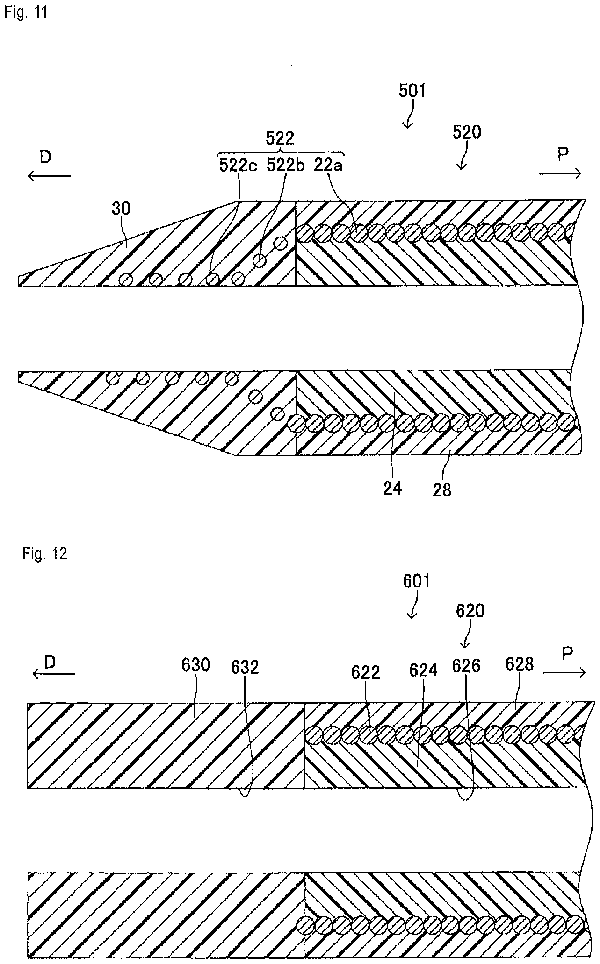

Conventionally, known is a catheter including a coil body configured such that an element wire or twisted wire is wound spirally to have a predetermined inner diameter, and a catheter shaft having an inner layer and an outer layer, the inner layer covering an inner peripheral surface of the coil body and having a hollow portion extending in an axis direction, the outer layer covering an outer peripheral surface of the coil body (see Patent Document 1). Since a catheter is to be inserted into a lumen such as a blood vessel, the digestive tract, and the urinary duct as well as into a body structure such as the thoracic cavity and the abdominal cavity, the catheter desirably includes a distal end portion (an end portion in the distal direction) having high flexibility. To this end, a resin tip may be joined to the distal end (the end face in the distal direction) of a catheter shaft to increase the flexibility of the distal end portion of the catheter. As an example of such a catheter, a catheter 601 is shown in Fig. 12. An arrow P represents the proximal direction, and an arrow D represents the distal direction. The catheter 601 includes a catheter shaft 620 having a coil body 622, an inner layer 624, and an outer layer 628; and a tip 630 joined to the distal end thereof. As shown in Fig. 12, a tip 630 includes a communication hole 632 in communication with a hollow portion 626 of the catheter shaft 620.

### CITATION LIST

### Patent Document

Patent Document 1: US Patent No. 6824553

### DISCLOSURE OF THE INVENTION

### Problems to be Solved by the Invention

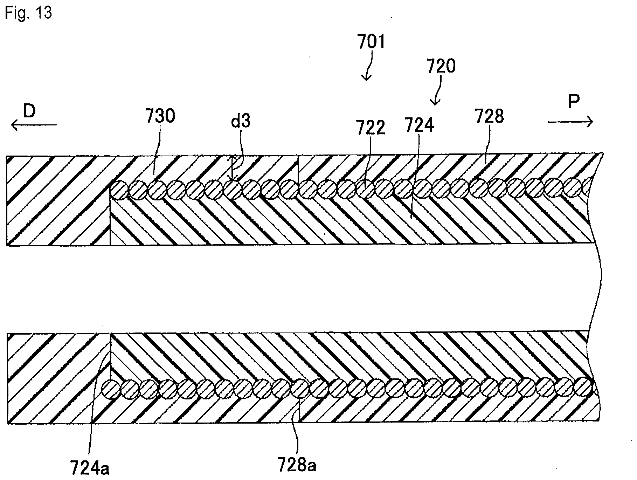

The catheter 601 has a problem in that the torque due to an operation performed by an operator may not efficiently be transmitted to the tip 630 (in other words, the torquability is low). That is, according to the catheter 601 where the coil body 622 is not arranged in the inside of the tip 630, the torque which is normally transmitted in the distal direction through the coil body can not be transmitted to the tip 630. Accordingly, a catheter 701 as shown in Fig. 13 has been proposed in which a coil body 722 and an inner layer 724 are extended through a tip 730 along the axis direction. According to the configuration of the catheter 701, the torque can be transmitted to the tip 730 through the coil body 722, leading to prevention of decreased torquability.

Meanwhile, a catheter desirably has a downsized tip (typically having a shape with a diameter decreasing toward the distal direction) for the purpose of improving followability. Downsizing of a tip may be performed, for example, by machining the tip. However, a configuration where the coil body 722 and the inner layer 724 are arranged in the inside of the tip 730 as in the catheter 701 has a relatively small thickness d3 (a length in the radial direction) of the tip 730 at a portion in which the coil body 722 and the inner layer 724 are arranged. This may not allow the tip 730 to be sufficiently downsized.

In order to solve the above problem, it is possible to remove the inner layer 724 arranged in the inside of the tip 730 and to decrease diameters of the tip 730 and the coil body 722 in the inside of the tip 730 by a length corresponding to the thickness of the inner layer 724 to be removed, thereby achieving downsizing of the tip 730. The above configuration makes it possible to downsize the tip 730 while preventing a decrease in torquability.

However, the above configuration may suffer from a decrease in a joining strength between the catheter shaft and the tip. That is, the tip 730 of the catheter 701 as shown in Fig. 13 is joined to a distal end 728a of an outer layer 728 and a distal end 724a of the inner layer 724 of the catheter shaft 720. In a configuration where the outer layer 724 is removed for downsizing of the tip 730, the proximal end of the tip is joined only to the distal end of the outer layer of the catheter shaft. As a result of this, a pulling force applied to the tip in the distal direction may result in breakage (detachment) of the tip from the catheter shaft at the joining region between the tip and the caterer shaft.

The present invention is made in order to solve the aforementioned problem. That is, an object of the present invention is to provide a catheter in which a tip can be downsized while preventing a decrease in torquability, and the joining strength between the tip and a catheter shaft can be further enhanced.

### Means for Solving the Problems

A catheter according to an embodiment of the present invention includes:
a catheter shaft including:
   a coil body being configured such that an element wire or twisted wire is wound spirally to have a first portion with a first inner diameter;
   an inner layer covering an inner peripheral surface of the first portion of the coil body and having a hollow portion extending in an axis direction; and
   an outer layer covering an outer peripheral surface of the first portion of the coil body; and
a tip made of a resin which is arranged at distal ends of the first portion of the coil body, the inner layer, and the outer layer and which has a communication hole in communication with the hollow portion of the inner layer, wherein,
the coil body further has a tapered portion disposed at the distal end of the first portion, the tapered portion having a diameter decreasing from the first inner diameter to a second inner diameter smaller than the first inner diameter,
the tapered portion of the coil body is arranged in the inside of the tip,
a gap is disposed between adjacent portions of the element wire at the tapered portion, and
the tip includes:
   an outer-layer joining region joined to the distal end of the outer layer; and
   an inner-layer joining region joined to the distal end of the inner layer,
   the outer-layer joining region being integrated with the inner-layer joining region through the gap.

### BRIEF DESCRIPTION OF THE DRAWINGS

Fig. 1 shows a schematic view of a catheter according to a first embodiment of the present invention.
Fig. 2 shows a sectional view of a part of a catheter shaft and a tip of the catheter according to the first embodiment of the present invention, the part of the catheter shaft and the tip being cut along a plane including the central axis.
Fig. 3 shows a sectional view along the III-III line of Fig. 2.
Fig. 4 shows a sectional view along the IV-IV line of Fig. 2.
Fig. 5 shows a sectional view of a part of a catheter shaft and a tip of a catheter according to a second embodiment of the present invention, cut along a plane including the central axis.
Fig. 6 shows a sectional view of a part of a catheter shaft and a tip of a catheter according to a variation of the second embodiment of the present invention, cut along a plane including the central axis.
Fig. 7 shows a sectional view of a part of a catheter shaft and a tip of a catheter according to a third embodiment of the present invention, cut along a plane including the central axis.
Fig. 8 shows a sectional view along the VIII-VIII line of Fig. 7.
Fig. 9 shows a sectional view of a part of a catheter shaft and a tip of a catheter according to a variation 1 of the third embodiment of the present invention, cut along a plane including the central axis.
Fig. 10 shows a sectional view along the X-X line of Fig. 9.
Fig. 11 shows a sectional view of a part of a catheter shaft and a tip of a catheter according to a variation 2 of the third embodiment of the present invention, cut along a plane including the central axis.
Fig. 12 shows a sectional view of a part of a catheter shaft and a tip of a conventional catheter, cut along a plane including the central axis.
Fig. 13 shows a sectional view of a part of a catheter shaft and a tip of another conventional catheter, cut along a plane including the central axis.

### PREFERRED MODE FOR CARRYING OUT THE INVENTION

### First Embodiment

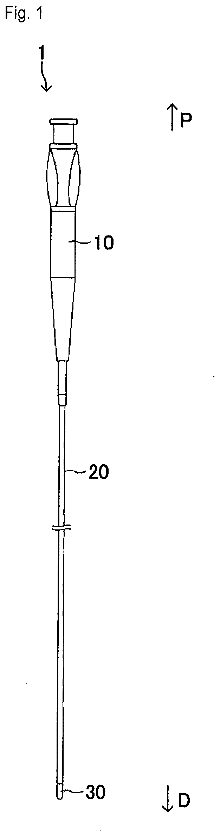

Below, a catheter 1 according to a first embodiment of the present invention will be described with reference to the drawings. A catheter is a flexible medical instrument which may be inserted into a lumen such as a blood vessel, the digestive tract, and the urinary duct as well as into a body structure such as the thoracic cavity and the abdominal cavity, and used for delivering a stent, an embolization coil, and the like to a lesion site, for injecting a chemical solution or a contrast agent, or for discharging a body fluid. Fig. 1 shows a schematic view of the catheter 1 according to the present embodiment. The catheter 1 is intended to be inserted into a blood vessel. As shown in Fig. 1, the catheter 1 is an elongated member, and includes a connector 10, a catheter shaft 20, and a tip 30. The connector 10 is connected to the proximal end of the catheter shaft 20. The tip 30 is located at the distal end portion of the catheter 1. It is noted that the catheter 1 intended to be inserted into a blood vessel is illustrated in the present embodiment, but the application of the catheter shall not be limited to this as long as the catheter includes a catheter shaft and a tip corresponding to the catheter shaft 20 and the tip 30, respectively.

The connector 10 is held and operated by an operator. When the operator operates the connector 10, the torque exerted by the operator is transmitted to the tip 30 through a coil body 22 described below. The connector 10 may also serve as a connection member for connecting various devices (for example, a three-way cock, a Y connector, and the like) to the catheter shaft 20.

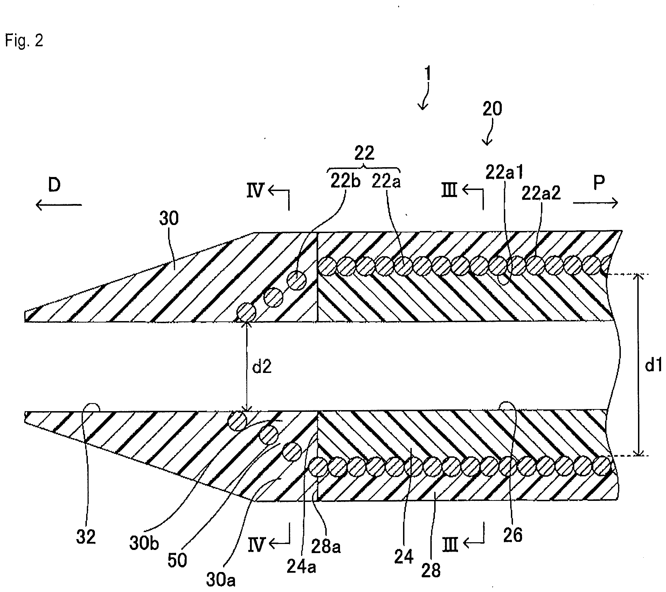

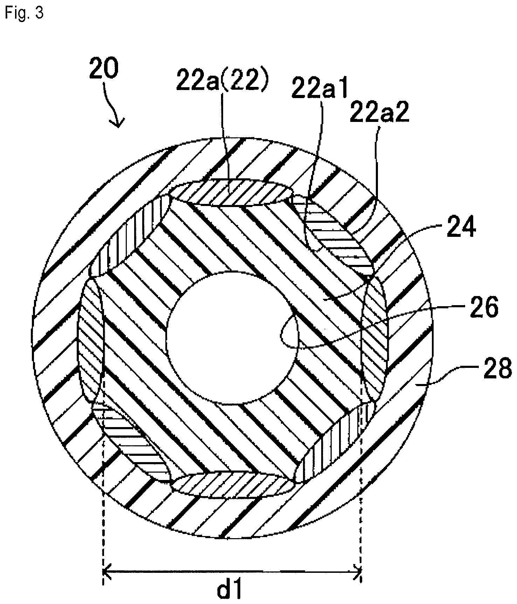

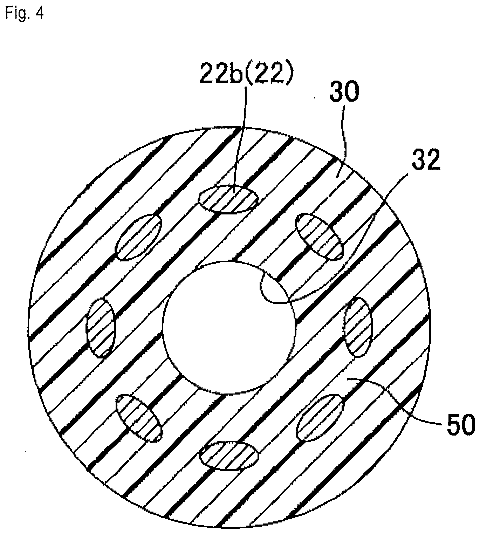

Fig. 2 shows a sectional view of a distal end portion of the catheter shaft 20 and the tip 30 cut along a plane including the central axis of the catheter 1. Fig. 3 shows a sectional view of the catheter shaft 20, cut along a plane orthogonal to the central axis of the catheter 1. Fig. 4 shows a sectional view of a portion where a tapered portion 22b (described below) of the tip 30 is located, cut along a plane orthogonal to the central axis of the catheter 1. As shown in Figs. 2 and 3, the catheter shaft 20 has the coil body 22, an inner layer 24, and an outer layer 28.

The coil body 22 is configured such that 8 metal element wires are wound spirally, and has a central axis coinciding with the central axis of the catheter 1. Each of the element wires has an element wire diameter which is uniform from one end through the other and the same among one another. Stainless steel is used as a metal material of each of the element wires according to the present embodiment, but the metal material of an element wire shall not be limited to this. For example, a Ni-Ti alloy may be used. Further, each of the element wires may be made of a different metal material to one another. It is noted that the number of element wires of the coil body 22 shall not be limited to 8, but may be appropriately selected depending on the shape and size of the coil body 22. Further, the coil body 22 may be made out of twisted wires in which a plurality of element wires are wound, instead of element wires.

As shown in Fig. 2, the coil body 22 has a first portion 22a with a substantially cylindrical shape, and the tapered portion 22b with a substantially truncated cone-like shape. The first portion 22a has a predetermined inner diameter d1. In the first portion 22a, the element wires are wound so that adjacent element wires are brought into contact with one another in the axis direction (see Fig. 2) and the circumferential direction (see Fig. 3). That is, there is no gap between adjacent element wires. Meanwhile, the tapered portion 22b is disposed at the distal end of the first portion 22a, and has a diameter decreasing toward the distal direction from the inner diameter d1 to a predetermined inner diameter d2 smaller than the inner diameter d1. It is noted that the inner diameter d1 corresponds to an example of the "first inner diameter", and the inner diameter d2 corresponds to an example of the "second inner diameter."

As shown in Figs. 2 and 4, the tapered portion 22b is arranged in the inside of the tip 30 (as described below). In the tapered portion 22b, the element wires are wound so that adjacent element wires are not brought into contact with one another in the axis direction (see Fig. 2) or the circumferential direction (see Fig. 4). That is, there is a gap 50 between adjacent element wires. The tapered portion 22b can, for example, be manufactured as follows. Specifically, a coil body with a substantially cylindrical shape which has the inner diameter d1 and extends along the axis direction in the distal direction from the distal end of the first portion 22a is swaged inwardly in the radial direction from the outside of the coil body. During this, a swaging force is increased toward the distal direction to shape the coil body into a substantially truncated cone-like shape having a diameter decreasing toward the distal direction. In addition, a force in the distal direction is applied to the coil body to form a gap between adjacent element wires. Subsequently, an unwanted portion is cut off from the distal end portion of the coil body. Thereby, the tapered portion 22b is manufactured.

The inner diameter of the coil body 22 refers to a diameter of a cross-section (a circle) of a hypothetical tubular member inscribed on the coil body 22 cut along a plane orthogonal to the axis direction. A portion of the hypothetical tubular member inscribed on the first portion 22a has a cylindrical shape, and a portion of the hypothetical tubular member inscribed on the tapered portion 22b has a truncated cone-like shape.

Here, when the coil body 22 includes a plurality of element wires as in the present embodiment as described above, the term "adjacent element wires" means two element wires adjacent in the axis direction and the circumferential direction among the plurality of element wires. On the other hand, the term "adjacent element wires" in a case of the coil body including a single element wire means "winding wires adjacent in the axis direction" in which a winding wire is defined as one turn of the element wire.

As shown in Figs. 2 and 3, the inner layer 24 is made of a resin, covers an inner peripheral surface 22a1 of the first portion 22a of the coil body 22 with a predetermined thickness, and also has a hollow portion 26 with a cylindrical shape extending in the axis direction at the center in the radial direction. The inner layer 24 entirely covers the inner peripheral surface 22a1 of the first portion 22a so that the first portion 22a of the coil body 22 is not exposed to the hollow portion 26. According to the present embodiment, polytetrafluoroethylene (PTFE) is used as a resin material of the inner layer 24, but the resin material of the inner layer 24 shall not be limited to this.

The outer layer 28 is made of a resin, and covers an outer peripheral surface 22a2 of the first portion 22a of the coil body 22 with a predetermined thickness. The outer layer 28 entirely covers the outer peripheral surface 22a2 of the first portion 22a so that the first portion 22a of the coil body 22 is not exposed to the outside of the outer layer 28. According to the present embodiment, a polyamide elastomer is used as a resin material of the outer layer 28, but the resin material of the outer layer 28 shall not be limited to this. Polyamides, polyesters, and the like may be used.

As shown in Fig. 2, the tip 30 is made of a resin, and disposed at the distal ends of the first portion 22a of the coil body 22, the inner layer 24, and the outer layer 28. The outer diameter of the tip 30 is substantially uniform at a proximal end portion (the end portion in a proximal direction P), and is substantially the same as the outer diameter at the distal end of the outer layer 28 of the catheter shaft 20. Further, a portion at the side of a distal direction D of the proximal end portion of the tip 30 has a diameter decreasing toward the distal direction D. This leads to downsizing of the tip 30. The tip 30 has a communication hole 32 with a cylindrical shape extending in the axis direction at the center in the radial direction. The communication hole 32 is in communication with the hollow portion 26 of the inner layer 24 of the catheter shaft 20, and has a diameter at the proximal end of the communication hole 32 which is substantially the same as a diameter at the distal end of the hollow portion 26. The communication hole 32 is located coaxially with the hollow portion 26, and their central axes coincide with the central axis of the catheter 1. The communication hole 32 and the hollow portion 26 constitute the inner cavity of the catheter 1.

The tip 30 is made of a resin material having a higher flexibility (i.e., a lower Young's modulus) and a lower melting point than the resin material(s) of the inner layer 24 and the outer layer 28. According to the present invention, polyurethane is used as a resin material of the tip 30, but the resin material of the tip 30 shall not be limited to this. A resin material having a higher flexibility and a lower melting point than the resin material(s) of the inner layer 24 and the outer layer 28 may be selected.

As described above, the tapered portion 22b of the coil body 22 of the catheter shaft 20 is arranged in the inside of the tip 30, and the gap 50 is disposed between adjacent element wires at the tapered portion 22b (see Figs. 2 and 4). A portion 30a of the tip 30 located at an outer periphery side of the tapered portion 22b is joined to a distal end 28a of the outer layer 28 of the catheter shaft 20. A portion 30b of the tip 30 located at an inner periphery side of the tapered portion 22b is joined to a distal end 24a of the outer layer 24 of the catheter shaft 20. Below, the portion 30a is called an "outer-layer joining region 30a", and the portion 30b is called an "inner-layer joining region 30b," accordingly. The outer-layer joining region 30a is integrated with the inner-layer joining region 30b through the gap 50.

Here, it is described a method of joining a resin (polyurethane) of the tip 30 to the distal end 28a of the outer layer 28 and the distal end 24a of the inner layer 24. Before the tip 30 is joined, the tapered portion 22b of the coil body 22 protrudes in the distal direction along the axis direction from the distal end 28a of the outer layer 28 and the distal end 24a of the inner layer 24 of the catheter shaft 20. At this stage, a cored bar having substantially the same size as the hollow portion 26 and the communication hole 32 is inserted through the coil body 22 at the center in the radial direction. While maintaining this state, a cylindrical polyurethane tube having an inner diameter larger than the diameter of the cored bar is fit into the outer periphery of the coil body 22. While maintaining a state where an end of the polyurethane tube is located in the vicinity of the distal end 28a of the outer layer 28 and the distal end 24a of the inner layer 24, the polyurethane tube is then heated and pressurized at a temperature at or above the melting point of polyurethane but lower than the melting points of the resin materials (PTFE and a polyamide elastomer) of the inner layer 24 and the outer layer 28. Then, melted polyurethane resin is joined to the distal end 28a of the outer layer 28, and enters into the inner periphery side of the tapered portion 22b through the gap 50 to be joined to the distal end 24a of the inner layer 24. A portion of the melted polyurethane resin joined to the distal end 28a of the outer layer 28 corresponds to the outer-layer joining region 30a, and a portion joined to the distal end 24a of the inner layer 24 corresponds to the inner-layer joining region 30b. Thus, the tip 30 is joined to the distal end 28a of the outer layer 28 and the distal end 24a of the inner layer 24. In other words, the tapered portion 22b is embedded in the inside of the tip 30. As understood from the above description, the outer-layer joining region 30a is integrated with the inner-layer joining region 30b, and there is no interface therebetween.

The catheter 1 according to the first embodiment has a configuration where a portion of the coil body 22 (the tapered portion 22b) is arranged in the inside of the tip 30. The configuration allows an operator to transmit a torque to the tip 30 through the coil body 22. This can prevent a decreased torquability due to the presence of the tip 30.

The inner layer 24 is not disposed in the inside of the tip 30, but the tapered portion 22b of the coil body 22 alone is disposed. The above tapered portion 22b has a shape having a diameter decreasing toward the distal direction by a length corresponding to the thickness of the inner layer 24. This configuration allows the tip 30 to be downsized to the extent that the diameter decreases in the tapered portion 22b, and thus this configuration can improve the followability of the catheter 1. In particular, for the catheter 1 intended to be inserted into a blood vessel (i.e., a catheter which will advance through a blood vessel along a guide wire), downsizing of the tip 30 can improve followability of the tip 30 with respect to a guide wire.

In addition, the tip 30 has not only the outer-layer joining region 30a joined to the distal end 28a of the outer layer 28 of the catheter shaft 20 but also the inner-layer joining region 30b joined to the distal end 24a of the inner layer 24 of the catheter shaft 20. Therefore, the joining strength of joining regions between the tip 30 and the catheter shaft 20 can be maintained at a level comparable to those of the conventional catheters 601, 701 (see Fig. 12 and Fig. 13) even when the inner layer 24 is removed from the tip 30 for the purpose of downsizing of the tip 30. The joining regions means ones between the outer-layer joining region 30a and the distal end 28a of the outer layer 28, and a joining region between the inner-layer joining region 30b and the distal end 24a of the inner layer 24. Additionally, it is noted that the joining strength between the tip 30 and the catheter shaft 20 can be more enhanced than those of the conventional catheters 601, 701 when the catheter 1 has a configuration as described below.

Specifically, a portion of the coil body 22 (i.e., the tapered portion 22b) embedded in the inside of the tip 30 has a substantially truncated cone-like shape having a diameter decreasing toward the distal direction, and each of the element wires of the tapered portion 22b continues from the corresponding element wire of the first portion 22a. According to this configuration, the inner-layer joining region 30b is caught up in the tapered portion 22b when a pulling force is applied to the tip 30 in the distal direction D. This can prevent the inner-layer joining region 30b from being pulled out to the distal direction side from the tapered portion 22b. In other words, the tapered portion 22b functions as an anchor for preventing the inner-layer joining region 30b from being pulled out to the distal direction side from the tapered portion 22b. The inner-layer joining region 30b is integrated with the outer-layer joining region 30a through the gap 50 disposed between adjacent element wires at the tapered portion 22b. This can prevent the inner layer joining region 30b from being pulled out to the distal direction side from the tapered portion 22b, which can, in turn, prevent breakage (detachment) of the tip 30 from a joining region with the catheter shaft 20. Thereby, the joining strength between the tip 30 and the catheter shaft 20 can be further enhanced as compared with the conventional catheters 601, 701.

According to the configuration of the catheter 1 of the first embodiment as described above, the tip 30 can be downsized while preventing decreased torquability, and the joining strength between the tip 30 and the catheter shaft 20 can be further enhanced.

Further, the tapered portion 22b can function as an anchor so that the inner-layer joining region 30b is caught up in the tapered portion 22b even if breakage (detachment) of the tip 30 from the catheter shaft 20 occurs at a joining region with the catheter shaft 20. This can prevent complete detachment of the tip 30 from the catheter shaft 20.

### Second Embodiment

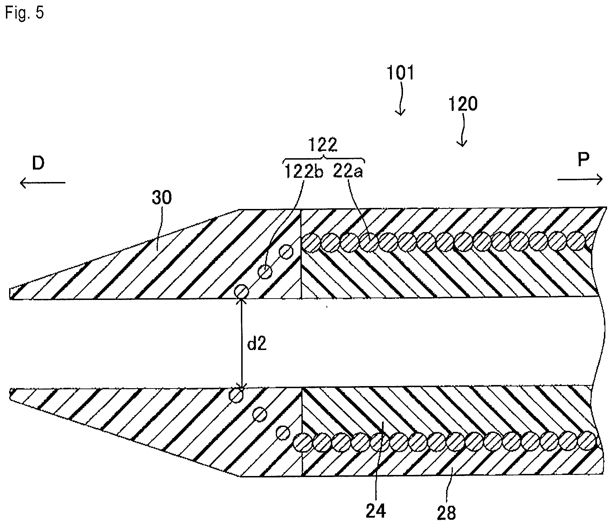

Next, it is described a catheter 101 according to a second embodiment of the present invention with reference to Fig. 5. Below, the same reference number is assigned to a member which has the same configuration as the catheter 1 according to the first embodiment, and the detailed description thereof will be omitted. The same applies to other embodiments and variations.

Fig. 5 shows a sectional view of a distal end portion of a catheter shaft 120 and the tip 30 cut along a plane including the central axis of the catheter 101. As shown in Fig. 5, the catheter 101 according to the present embodiment differs from the catheter 1 according to the first embodiment in that each of the element wires of a tapered portion 122b of a coil body 122 has an element wire diameter smaller than the element wire diameter of each of the element wires of the first portion 22a. The tapered portion 122b can be manufactured as follows. Before the tip 30 is joined to the outer layer 28 and the inner layer 24 of the catheter shaft 120, the tapered portion 22b of the catheter 1 according to the first embodiment may be immersed in an electrolytic solution to decrease the diameter of each of the element wires of the tapered portion 22b by electrolytic polishing. It is noted that strictly speaking, electrolytic polishing is performed under a condition where the tapered portion 22b is designed to have an inner diameter slightly smaller than d1 at the proximal end thereof, and also have an inner diameter slightly smaller than d2 at the distal end thereof, considering the amount to be polished by electrolytic polishing. This also applies to other embodiments and variations in which the diameter of a tapered portion is decreased by electrolytic polishing. By the process as described above, the tapered portion 122b after electrolytic polishing has a shape having the inner diameter d1 at the proximal end thereof and the inner diameter d2 at the distal end thereof. Alternatively, the tapered portion 122b can also be manufactured by grinding the tapered portion 22b of the catheter 1 according to the first embodiment with a rotary grindstone.

The above configuration can also show similar operational effects as the catheter 1 according to the first embodiment. In addition, the above configuration, where each of the element wires of the coil body 122 disposed in the inside of the tip 30 (i.e., the tapered portion 122b) has an element wire diameter smaller than the element wire diameter of each of the element wires of the first portion 22a, can improve the flexibility of the tip 30 while maintaining a good torquability of the tip 30.

### Variations

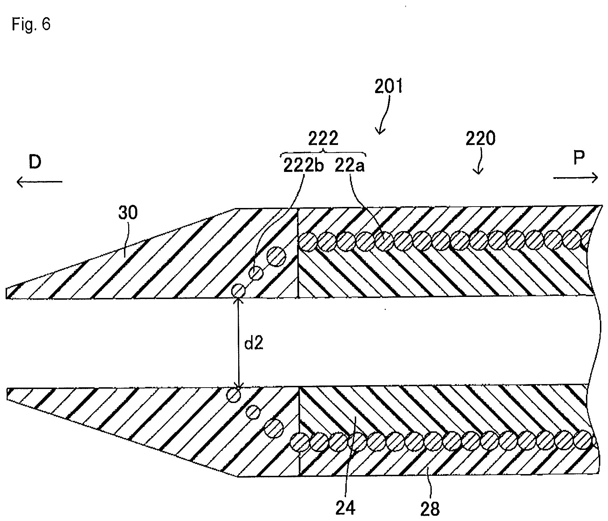

Next, a catheter 201 according to a variation of the second embodiment of the present invention will be described with reference to Fig. 6. Fig. 6 shows a sectional view of a distal end portion of the catheter shaft 220 and the tip 30 cut along a plane including the central axis of the catheter 201. As shown in Fig. 6, the catheter 201 according to the present variation differs from the catheter 101 according to the second embodiment in that only the element wires of the distal end portion of a tapered portion 222b of a coil body 222 each has an element wire diameter smaller than the element wire diameter of each of the element wires of the first portion 22a. Before the tip 30 is joined to the outer layer 28 and the inner layer 24 of the catheter shaft 220, each of the element wires at the distal end portion of the tapered portion 22b of the catheter 1 according to the first embodiment may be subjected to electrolytic polishing for decreasing the diameter thereof, thereby manufacturing the tapered portion 222b. Alternatively, the tapered portion 222b can also be manufactured by grinding the distal end portion of the tapered portion 22b of the catheter 1 according to the first embodiment with a rotary grindstone.

The above configuration can also show similar operational effects as the catheter 1 according to the first embodiment. In addition, the above configuration, where each of the element wires of the distal end portion of the tapered portion 222b has an element wire diameter smaller than the element wire diameter of each of the element wires of the first portion 22a, can improve the flexibility of the tip 30 at a portion located in the vicinity of the distal end portion of the tapered portion 222b as compared with the configuration of the catheter 1 according to the first embodiment while maintaining a good torquability of the tip 30.

It is noted that the diameter of each of the element wires of the distal end portion of the tapered portion 222b is decreased in the present variation, but the element wire diameter of each of the element wires of a different portion of the tapered portion 222b may be decreased. That is, the element wire diameter of each of the element wires of at least a portion of the tapered portion 222b may be set to be smaller than the element wire diameter of each of the element wires of the first portion 22a. The above configuration can improve the flexibility of the tip 30 at a portion located in the vicinity of a portion where the diameter of each of the element wires of the tapered portion 222b is decreased, while maintaining a good torquability of the tip 30. Further, in a case where the coil body 222 includes a plurality of element wires, all of the plurality of element wires of at least a portion of the tapered portion 222b may not necessarily have decreased diameters. A configuration where only some of the plurality of element wires have decreased diameters may be used.

### Third Embodiment

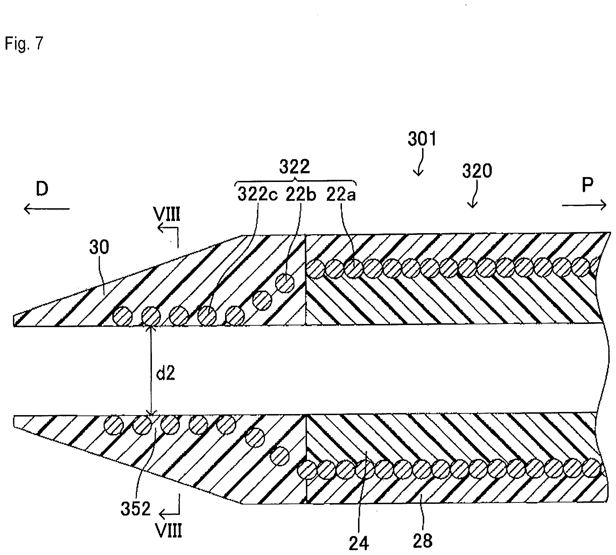

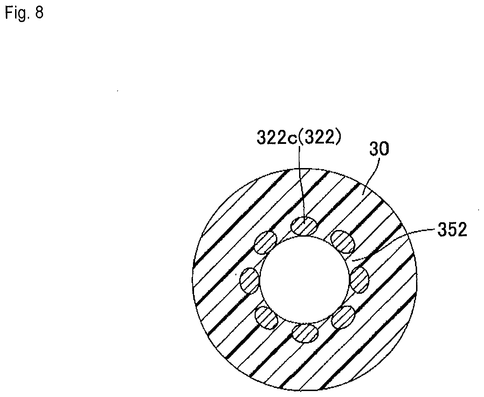

Next, a catheter 301 according to a third embodiment of the present invention will be described with reference to Figs. 7 and 8. Fig. 7 shows a sectional view of a distal end portion of a catheter shaft 320 and the tip 30, cut along a plane including the central axis of the catheter 301. Fig. 8 shows a sectional view of a portion where a second portion 322c (described below) of the tip 30 is located, cut along a plane orthogonal to the central axis of the catheter 301. As shown in Fig. 7, the catheter 301 according to the present embodiment differs from the catheter 1 according to the first embodiment in that a coil body 322 has the second portion 322c disposed at the distal end of the tapered portion 22b. The second portion 322c has a substantially cylindrical shape extending in the axis direction, and has an inner diameter of d2 (i.e., has an inner diameter having the same value as the inner diameter at the distal end of the tapered portion 22b). In the second portion 322c, element wires are wound so that adjacent element wires are not brought into contact with one another in the axis direction (see Fig. 7) or the circumferential direction (see Fig. 8). That is, a gap 352 is disposed between adjacent element wires. The second portion 322c can be manufactured as follows. After the tapered portion 22b is manufactured according to the aforementioned method, a portion of the coil body located at the distal end side of the tapered portion 22b may be swaged with a force which is constant throughout the axis direction. A force in the distal direction may also be applied to the portion of the coil body to form a gap between adjacent element wires. Subsequently, an unwanted portion is cut off from the distal end portion of the coil body.

The above configuration can also show similar operational effects as the catheter 1 according to the first embodiment. In addition, since the above configuration where the second portion 322c is disposed at the distal end of the tapered portion 22b makes it possible to transmit suitably the torque to the distal end side of the tip 30, it is possible to improve torquability. Further, the gap 352 disposed between adjacent element wires at the second portion 322c can reduce a likelihood of mutual interference between adjacent element wires at the second portion 322c when a force is applied to the tip 30. This can prevent a decrease in the flexibility of the tip 30 due to the presence of the second portion 322c in the inside of the tip 30.

### Variation 1

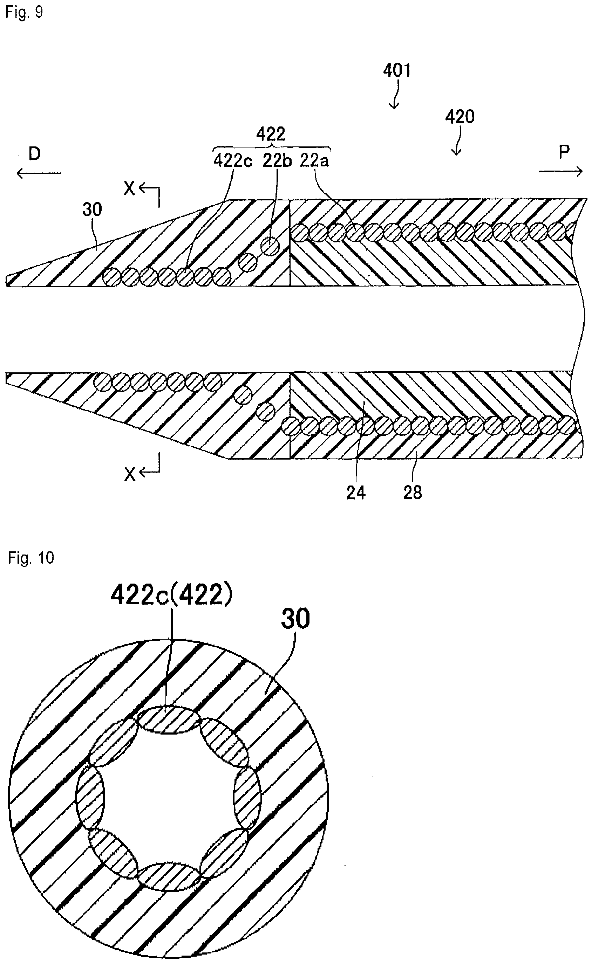

Next, a catheter 401 according to a variation 1 of the third embodiment of the present invention will be described with reference to Figs. 9 and 10. Fig. 9 shows a sectional view of a distal end portion of a catheter shaft 420 and the tip 30 cut along a plane including the central axis of the catheter 401. Fig. 10 shows a sectional view of a portion where a second portion 422c (described below) of the tip 30 is located, the portion being cut along a plane orthogonal to the central axis of the catheter 401. As shown in Fig. 9, the catheter 401 according to the present variation differs from the catheter 301 according to the third embodiment in that element wires are wound so that adjacent element wires are brought into contact with one another in the axis direction (see Fig. 9) and the circumferential direction (see Fig. 10) in the second portion 422c of the coil body 422, i.e., a gap is not disposed between adjacent element wires.

The above configuration can also show similar operational effects as the catheter 1 according to the first embodiment. In addition, since the above configuration where the second portion 422c is disposed at the distal end of the tapered portion 22b makes it possible to transmit suitably the torque to the distal end side of the tip 30, it is possible to improve torquability.

### Variation 2

Next, a catheter 501 according to a variation 2 of the third embodiment of the present invention will be described with reference to Fig. 11. Fig. 11 shows a sectional view of a distal end portion a catheter shaft 520 and the tip 30, cut along a plane including the central axis of the catheter 501. As shown in Fig. 11, the catheter 501 according to the present variation differs from the catheter 301 according to the third embodiment in that the element wire diameter of each of the element wires of a tapered portion 522b and a second portion 522c of a coil body 522 is smaller than the element wire diameter of each of the element wires of the first portion 22a of the coil body 522. The tapered portion 522b and the second portion 522c can be manufactured by decreasing the diameter of each of the element wires of the tapered portion 22b and the second portion 322c of the catheter 301 according to the third embodiment by electrolytic polishing before the tip 30 is joined to the outer layer 28 and the inner layer 24 of the catheter shaft 520. Alternatively, the tapered portion 522b and the second portion 522c can also be manufactured by grinding the tapered portion 22b and the second portion 322c of the catheter 301 according to the third embodiment with a rotary grindstone.

The above configuration can also show similar operational effects as the catheter 301 according to the third embodiment. In addition, the element wire diameter of each of the element wires of the tapered portion 522b and the second portion 522c of the catheter 501 according to the present variation is smaller than the element wire diameter of each of the element wires of the tapered portion 22b and the second portion 322c of the catheter 301 according to the third embodiment. This configuration can further enhance the flexibility of the tip 30 as compared with the catheter 301.

The catheters according to the embodiments and variations have been described above, but the present invention shall not be limited to these embodiments and variations. It is possible to make various alterations without departing from the purposes of the present invention.

For example, each of the element wires of the coil body 22 is composed of a single continuous solid wire in the aforementioned embodiments and variations, but may alternatively be a single element wire in which a plurality of solid wires made of different materials are joined to one another via their end faces. For example, the end face of a platinum solid wire may be joined to the end face of a stainless-steel solid wire to form a single element wire. In that case, a platinum portion being arranged at the distal end side of the coil body 22 can allow the front end portion (the distal end portion) of the catheter 1 to be clearly captured in a roentgenogram.

A joining site at which a plurality of solid wires are joined to form an element wire, if such an element wire is formed, is preferably not located at a position in the vicinity of the boundary between the first portion 22a and the tapered portion 22b. This is because a joining site located at a position in the vicinity of the boundary might cause fracture and disconnection of the coil body at that site, resulting in breakage (detachment) of the tip 30 from the catheter shaft 20. However, the joining site may be located at a position in the vicinity of the boundary in a case where the joining strength of the joining site is higher than the tensile strength of the tip 30.

In a case where the coil body 22 includes a plurality of element wires, the element wire diameters of the element wires may be different to one another. Moreover, a configuration may be used in which adjacent element wires are partially brought into contact with one another in either the axis direction or the circumferential direction at the tapered portion 22b without a gap disposed at that position.

The catheter shaft 20 may further include a braid as a reinforcement body. The braid is a metal member having a substantially cylindrical shape, and arranged inside the inner layer 24 so that the central axis thereof coincides with that of the catheter 1. When the braid is arranged, the circularity on a plane orthogonal to the central axis of the catheter shaft 20 can be suitably maintained. It is noted that the braid may be arranged in the inside of the tip 30.

A gap between adjacent element wires may also be disposed at the first portion 22a of the coil body 22. In addition, the coil body 22 shall not be limited to a metal product, but may be made of a resin.

The resin material of the outer-layer joining region 30a of the tip 30 may not necessarily be same as the resin material of the inner-layer joining region 30b. For example, polyurethane may be used for the inner-layer joining region 30b while a material in which tungsten powder is kneaded with polyurethane may be used for the outer-layer joining region 30a. Even in this case, the outer-layer joining region 30a is integrated with the inner-layer joining region 30b through the gap 50 and there exists no interface between them.

## Claims

1. A catheter comprising:
a catheter shaft including:
a coil body being configured such that an element wire or twisted wire is wound spirally to have a first portion with a first inner diameter;
an inner layer covering an inner peripheral surface of the first portion of the coil body and having a hollow portion extending in an axis direction; and
an outer layer covering an outer peripheral surface of the first portion of the coil body; and
a tip made of a resin which is arranged at distal ends of the first portion of the coil body, the inner layer, and the outer layer and which has a communication hole in communication with the hollow portion of the inner layer, wherein,
the coil body further has a tapered portion disposed at the distal end of the first portion, the tapered portion having a diameter decreasing from the first inner diameter to a second inner diameter smaller than the first inner diameter,
the tapered portion of the coil body is arranged in the inside of the tip,
a gap is disposed between adjacent portions of the element wire at the tapered portion, and
the tip includes:
an outer-layer joining region joined to the distal end of the outer layer; and
an inner-layer joining region joined to the distal end of the inner layer,
the outer-layer joining region being integrated with the inner-layer joining region through the gap.

2. The catheter according to claim 1, wherein
the element wire diameter of an element wire of at least a portion of the tapered portion of the coil body is smaller than the element wire diameter of an element wire of the first portion.

3. The catheter according to claim 1, wherein
the element wire diameter of an element wire of the tapered portion of the coil body is smaller than the element wire diameter of an element wire of the first portion.

4. The catheter according to any one of claims 1 to 3, wherein
the coil body further includes a second portion disposed at a distal end of the tapered portion and having the second inner diameter,
a gap is disposed between adjacent portions of the element wire at the second portion of the coil body.
